# EUROPEAN PATENT SPECIFICATION

(11) **EP 3 404 614 B1**
(45) Date of publication and mention of the grant of the patent: **26.02.2020**
(21) Application number: 17171000.7
(22) Date of filing: 15.05.2017
(51) Int. Cl.: G06T 7/55

(54) **METHOD FOR RECONSTRUCTING A RECONSTRUCTION DATA SET OF A VESSEL SEGMENT, IMAGING DEVICE, COMPUTER PROGRAM, AND STORAGE MEDIUM**
VERFAHREN ZUR REKONSTRUKTION EINES REKONSTRUKTIONSDATENSATZES EINES GEFÄSSSEGMENTS, BILDGEBUNGSVORRICHTUNG, COMPUTERPROGRAMM UND SPEICHERMEDIUM
PROCÉDÉ DE RECONSTRUCTION D'UN ENSEMBLE DE DONNÉES DE RECONSTRUCTION D'UN SEGMENT DE VAISSEAU, DISPOSITIF D'IMAGERIE, PROGRAMME INFORMATIQUE ET SUPPORT D'INFORMATIONS

(43) Date of publication of application: 21.11.2018
(73) Proprietor: Siemens Healthcare GmbH, 91052 Erlangen (DE)
(72) Inventor: Bauer, Sebastian, 91054 Erlangen (DE); Berger, Martin, 91052 Erlangen (DE); Redel, Thomas, 91099 Poxdorf (DE); Lauritsch, Günter, 90427 Nürnberg (DE); Preuhs, Alexander, 91058 Erlangen (DE)

(56) References cited:
- WO-A1-01/85030
- US-A1- 2015 339 847

## Description

The invention concerns a method for at least symbolically reconstructing a reconstruction data set of at least one vessel segment in a vessel tree of a patient, wherein input data for the reconstruction comprises at least two two-dimensional angiographic projection images taken in different acquisition geometries. The invention also concerns an imaging device, a computer program and an electronically readable storage medium.

It is already known that higher dimensional data sets can be reconstructed from at least two lower dimensional data sets, providing a view from different projection directions. For example, three-dimensional data sets can be reconstructed from two-dimensional projection images. If only sparse information in projection space is available, for example in the case of only a few projection images, it is known to only symbolically reconstruct objects, i.e. not calculating attenuation values for each voxel, but only for example boundaries and/or centerlines of objects. Such reconstruction data sets however, at least provide information on the spatial position and extension of the objects.

Reconstruction, in particular from sparse sets of projection images, is often used in diagnostic examinations of blood vessels. Lesions, for example stenosis, can be reconstructed from angiographic projection images. Usually, a certain vessel segment is of interest, which can, for example, be marked/manually segmented in the projection images after a few of them have been acquired. However, the acquired projection images are often not optimally suited for reconstruction, for example regarding overlapping vessels and/or foreshortening, leading to reconstruction errors and thus uncertainties in evaluating the data.

An exemplary field of application of such examinations are stenosis in the coronary arteries. To treat arteries constricted by atheromatous plaque, stents are used to restore blood flow. As there are also risks associated with minimally invasive insertion of such stents, only hemodynamically relevant stents are treated. The hemodynamic relevance of a stenosis can be assessed by measuring the fractional flow reserve (FFR). While it is known, for example from an article by Tonino et al., "Fractional Flow Reserve versus Angiography for Guiding Percutaneous Coronary Intervention", New England Journal of Medicine 360(3), pp. 213-224, 2009, to invasively measure the FFR, it has recently been proposed to measure the FFR non-invasively, i.e. virtually.

The non-invasive, virtual measurement of FFR provides a secure, fast and inexpensive alternative to a measurement by pressure wire, see for example the article by Monique Tröbs et al., "Comparison of Fractional Flow Reserve Based on Computational Fluid Dynamics Modeling Using Coronary Angiographic Vessel Morphology Versus Invasively Measured Fractional Flow Reserve", The American Journal of Cardiology 117 (1), pp. 29-35, 2016. The symbolical reconstruction data set in this case serves as a three-dimensional model, on which computational fluid dynamics (CFD) is performed. The accuracy of the reconstruction is important, since it directly affects the calculation if the FFR and thus the treatment decision.

Reconstruction accuracy is affected by multiple factors. Firstly, an accurate segmentation of the vessels in the two-dimensional angiographic projection images is necessary. Secondly, the geometric correlation of the individual angiography scenes has to be well estimated, which can be impeded by heart and respiratory movement and inaccurate geometrical calibration of the imaging device. Finally, the choice of projection directions plays a decisive role. Here, angiographic projection images having a projection direction perpendicular to the vessel segment and a sufficient angle difference to each other are advantageous.

Known reconstruction techniques are based on retrospectively manually choosing suitable angiographic projection images from a preceding acquisition. The acquisition geometries chosen by the medical staff are often not optimal for reconstruction. For example, overlapping vessels may impede segmentation. Furthermore, perspectively shortened (foreshortened) vessel depiction as well as inadequate sets of projection angles may complicate reconstruction.

Today's workflow is based on using already acquired angiographic projection images and selecting a subset to reconstruct the data set, for example a three-dimensional model. As an example, it is referred to "IZ3D" reconstruction software, distributed by the applicant. The disadvantage of these systems is that the user has to work with data already acquired in acquisition geometries not optimized for reconstruction or acquire additional angiographic projection images, increasing radiation and contrast agent exposure. Additionally, in this workflow, the sequential angiographic projection scenes are acquired independently from each other. In this process, information may be lost.

It has been proposed in not yet laid open patent application DE 10 2016 210 003.3 to acquire additional angiographic projection images in predetermined angular increments along a circular trajectory defined by a rotation axis. The rotation axis is estimated from a first, single angiographic projection image.

WO 01/85030 A1 discloses a method and system for imaging an artery contained in an arterial tree. Here, a microprocessor generates a three-dimensional reconstruction of the arterial tree from two or more angiographic images obtained from different perspectives.

US 2015/0339847 A1 discloses a method for vascular modelling. Here, projection and/or image registration is iteratively altered to improve feature position matching.

It is an object of the current invention to provide a method supplying better input data for a reconstruction and thus a qualitatively improved reconstruction data set.

This object is achieved by a method according to claim 1, an imaging device according to claim 12, a computer program according to claim 13 and an electronically readable storage medium according to claim 14.

An inventive method for at least symbolically reconstructing a reconstruction data set of at least one vessel segment in a vessel tree of a patient, wherein input data for the reconstruction comprises at least two two-dimensional angiographic projection images acquired in different acquisition geometries, comprises the following steps:
- acquiring at least one first angiographic projection image showing the vessel segment,
- automatically determining an evaluation measure for each first angiographic projection image using a three-dimensional preliminary information on the vessel segment, the evaluation measure describing the suitability of the at least one angiographic projection image for reconstructing the reconstruction data set,
- if a quality criterion evaluating the evaluation measure is not fulfilled, determining at least one additional acquisition geometry using the three-dimensional preliminary information and/or the evaluation measure and acquiring, in each additional acquisition geometry, at least one second angiographic projection image,
- reconstructing the reconstruction data set from the at least one second angiographic projection image and/or at least one of the at least one first angiographic projection image fulfilling a suitability criterion evaluating the evaluation measure.

It is proposed to use three-dimensional preliminary knowledge to evaluate the suitability of angiographic projection images and, in case more data is needed, additional acquisition geometries. The modified workflow thus comprises a step wherein the already acquired first angiographic projection images are evaluated. This may be only one image, but will often be a set of projection images, as is also true for the second angiographic projection images, as further explained below. It is noted that not necessarily each first angiographic projection image is evaluated on its own. Quite the contrary, in many cases evaluation measures will apply to multiple angiographic projection images. Thus, each first angiographic projection image is assigned at least one evaluation measure. The quality criterion checks if enough first angiographic projection images (or at least one group of them) fulfil the suitability criterion to reconstruct solely from them in sufficient quality; if it is not fulfilled, second angiographic projection images are acquired to supplement the first angiographic projection images or to even provide all angiographic projection images used for reconstruction if all first angiographic projection images are not suitable to be used, at least not if a certain data quality of the reconstruction data set is aimed at. This approach results in an optimized selection of first angiographic projection images to use in reconstructing and/or, if applicable, in an optimized planning and acquisition of second angiographic projection images. The user can be guided during the examination and an improved foundation for the three-dimensional reconstruction is supplied.

Compared with a pure retrospective selection of angiographic projection images to use for reconstruction, a guided and automatically planned acquisition of additional second angiographic projection images allows to include the information from the first angiographic projection images, as opposed to independent acquisition of scenes.

Generally, the quality of the reconstructed vessel segment/vessel tree can be strongly improved and kept at a constant high level over multiple patients and examinations. The automatic planning of additional acquisitions results in a faster examination procedure, in particular while also improving diagnostic quality. The necessary radiation exposure and the amount of contrast agent can be reduced since a minimal number of acquisitions can be achieved.

The three-dimensional preliminary information may in particular describe at least the three-dimensional course and/or position and orientation of the vessel segment. It can be approximate and/or based on assumptions. Preferably, the preliminary information also describes additional vessels in the vessel tree, in particular all vessels. For example, the preliminary information can comprise centerlines of the vessel segment and/or further segments/vessels in the vessel tree.

Each angiographic projection image may be derived from or be part of a scene which comprises multiple images acquired at different times.

As will be further discussed below, evaluation measures and criteria can be applied or relate to single or multiple angiographic projection images. For example, regarding difference in projection angles, pairs or groups of angiographic projection images/acquisition geometries can be evaluated. Usually, at least the quality criterion will check if at least one group of first angiographic projection images can be found among the first angiographic projection images which allows a reconstruction of a quality aimed at, in particular exceeding a threshold.

It is noted that the method may also be applied to multiple vessel segments, wherein an optimal solution for all these vessel segments is searched. For example, evaluation measures can be calculated for all vessel segments and the quality criterion may take into account all of them. It is also possible to choose the acquisition geometries for the second angiographic projection images such that the minimal number of second angiographic projection images is acquired, reducing radiation exposure for the patient.

Multiple sources of helpful three-dimensional preliminary information exist, which can be used in the current invention. In preferred embodiments, the three-dimensional preliminary information comprises, or is derived from, a previously acquired three-dimensional image data set, in particular a CT data set, which is registered to the imaging device acquiring the angiographic projection images, and/or the three-dimensional preliminary information is determined by preliminary reconstruction from, in particular the at least two first, angiographic projection images, and/or the three-dimensional preliminary information is derived from one two-dimensional first angiographic projection image and at least one assumption on how the acquisition geometry of the first angiographic projection image was chosen.

As a first option, the three-dimensional preliminary information may comprise or be derived from a previously acquired three-dimensional image data set of the patient. The image data set can, for example, be a computer tomography data set showing the vessel tree. Using techniques of 2D-3D-registration, for example by registering at least one of the at least one first angiographic projection image to the image data set, the three-dimensional preliminary information can also be used to optimize the reconstruction in a following angiographic examination. Of course, also preliminary information derived from such an image data set, for example a model of the vessel tree, can be used by registering the underlying image data set or the derived information to the angiographic projection images.

In a second option, three-dimensional preliminary information can also be derived from the first angiographic projection images or other available angiographic projection images. A preliminary reconstruction can be performed to derive the three-dimensional preliminary information. It is again noted that such preliminary reconstruction does not need to have a high quality, but mainly serves as a guide for approximate vessel courses. The first angiographic projection images may be used, but it is also possible, to use so-called standard projections acquired from the patient in the course of diagnostics. Alternatively, one or more acquisitions from a so-called heart sweep may also be used for this preliminary reconstruction. As already explained, the requirements for a sufficiently accurate 3D-reconstruction do not have to be met.

If only one two-dimensional view, for example one two-dimensional first angiographic projection image or scene, is available, it is nonetheless also possible to derive three-dimensional preliminary information. For example, it can be assumed that the vessel segment of interest lies inside or close to the isocenter and/or the projection direction is perpendicular to the course of the vessel segment. Using such assumptions, the three-dimensional orientation of the vessel segment can be estimated, as for example described in the already cited DE 10 2016 210 003.3.

The evaluation measure for one first angiographic projection image is determined depending on foreshortening of the vessel segment and/or overlap with other vessels occurring in the acquisition geometry of the evaluated first angiographic projection image. The evaluation measure may thus describe the quality of the acquisition parameters of an acquisition geometry used for a first angiographic projection image. In a certain angiographic projection image, it can be assessed if the projection direction favours foreshortening and/or vessel overlap. Both effects would be detrimental in reconstruction, the more they are present. In a concrete embodiment, a foreshortening value is determined by comparing a direction of the vessel segment according to the three-dimensional preliminary information and an image plane according to the acquisition geometry of the first angiographic projection image. Optimally, the direction of the vessel segment is perpendicular to the projection direction. A preferred overlap measure will be discussed below as it is applicable for both evaluation and determination of additional acquisition geometries.

Preferably, the evaluation measure for a pair of first angiographic projection images is determined depending on the relative projection angle of the acquisition geometries. The difference in projection angles for a pair of first angiographic projection images is important when assessing these images with respect to reconstruction. Ideally, the difference in projection angles is 90 ° if two angiographic projection images are to be used for reconstruction. This means that the projection directions of these images are optimally perpendicular.

In an especially preferred embodiment, to determine the evaluation measure and/or the additional acquisition geometry, an overlap measure for an acquisition geometry is calculated by
- forward projecting the three-dimensional preliminary information to determine a first virtual projection image relating to the vessel segment of interest and a second virtual projection image relating to other vessels in the vessel tree,
- deriving a distance weighing image from the first virtual projection image, comprising a weight decreasing outward from the boundary of the vessel segment in the first virtual projection image,
- measuring a proximity of other vessels by multiplying the distance weighing image and the second virtual projection image and measuring an overlap by multiplying the first virtual projection image and the second virtual projection image,
- deriving the overlap measure by combining the multiplication results.

Using these steps, an overlap measure (overlap metric) can not only be determined automatically, but also includes adjacency in addition to actual overlap. The closer another structure is to the vessel segment of interest, the more likely it is that segmentation is impeded or error-prone. It is noted that this determination of an overlap measure may also be used independently from the above-described angiographic workflow. It is conceivable to provide a method for determining at least one acquisition geometry for acquiring angiographic projection images of a vessel segment, wherein a circular trajectory perpendicular to the vessel segment according to three-dimensional preliminary information is determined and wherein for different acquisition geometries along the trajectory overlap measures are calculated as described above and acquisition geometries to be used to acquire angiographic projection images are determined based on the overlap measure, in particular such that acquisition geometries having a lower overlap measure are preferred. In this manner, an automatic determination of suitable projection directions along the trajectory is possible, which, in the scope of the workflow according to the invention, can be used for evaluating acquisition geometries of already acquired first angiographic projection images and for determining additional acquisition geometries for second angiographic projection images.

It is noted that forward projecting three-dimensional preliminary information to create virtual projection images has already been described, for example by W. Sato et al. in "A viewpoint determination system for stenosis diagnosis and quantification in coronary angiographic image acquisition", IEEE transactions on medical imaging, Vol. 17, No. 1, pages 121 to 137, 1998. However, in the context of foreshortening and vessel overlap, these images have only been displayed to medical staff for further evaluation.

According to this embodiment of the invention, however, an automatic determination of an overlap measure is performed, taking into account actual overlap as well as proximity of other vessels depicted close to the vessel segment of interest. To measure this proximity, a distance weighing image is calculated from the first virtual projection image, such that the boundaries of the vessel segment in the first virtual projection image are determined and a weight is assigned to each pixel outwards from the boundary, decreasing in value away from the boundary. The depiction of the vessel segment itself inside the boundary, however, is assigned a weight of zero. Also, areas distanced from the boundary by more than a distance limit can also be assigned a weight of zero as image value. If a so-defined distance weighing image and the second virtual projection image are multiplied pixel by pixel, only structures close to the vessel segment in the first virtual projection image are maintained, in particular weighted stronger if they are closer to the boundary.

Multiplying the first and second virtual projection images pixel by pixel results in maintaining only overlapping vessel parts, as the first virtual projection image only shows the vessel segment, the second virtual projection image only shows the background, i.e. the other vessels of the vessel tree.

The two multiplication result images can now be combined to derive the overlap measure, for example by adding the multiplication result images up and summing over the pixels of the summary result image. It is possible to assign weighing factors to each multiplication result image when adding them to the summary result image. In this manner, actual overlap and adjacency can be differently weighted, which can, however, also be achieved by choosing the weights in the distance weighing image accordingly. The overlap measure can be normalized, for example by dividing the sum over all pixels of the first virtual projection image.

Preferably, independent of the actual format of the three-dimensional preliminary information, the first virtual projection image and the second virtual projection image are determined as binary images, simplifying the determination process as described above. An image value is zero if no vessel is present and one otherwise. The virtual projection images may also be confined to a vicinity of the vessel segment.

Returning to general preferred embodiments of the method according to the invention, it can be provided that, to determine the evaluation measure and/or the additional acquisition geometry, at least one eccentricity of the vessel segment and/or the visibility of at least one bifurcation in the vessel segment are taken into account. An eccentrically deformed vessel, for example due to stenosis, may appear to have different diameters when imaged with different acquisition geometries. If the eccentric shape of the vessel segment, however, is at least approximately known, it is possible to assess the helpfulness of information derived in a certain acquisition geometry. If, for example, two angiographic projection images of an approximately elliptical vessel segment are each taken at 45 ° between the long axis and the short axis, the same diameter will be measured and the vessel segment will be assumed circular, such that these acquisition geometries will result in less useful information as, for example, acquisition geometries with a projection direction essentially along the long or short axis of the elliptic shape.

Also of relevance can be bifurcations along the vessel segment which are to be measured accurately. While in some acquisition geometries, due to overlap, the bifurcation may not or not accurately be visible, other acquisition geometries provide a good view on the actual position of the ostium. Thus, the presence of bifurcations can also be taken into account when evaluating the suitability of angiographic projection images for reconstruction.

As already explained, depending on the evaluation results, in particular the fulfilment of the quality criteria, the acquisition of further angiographic projection images can result in a considerate improvement of quality of the reconstruction. To plan these acquisition geometries, also the three-dimensional preliminary information regarding the vessel segment/vessel tree is used to define the number and acquisition geometry of second angiographic projection images. The calculated evaluation measures can be taken into account here and/or also be calculated for candidate acquisition geometries. It is noted that also an optimal number of additional acquisition geometries may be determined.

Preferably, when determining additional acquisition geometries, also the movement range of the imaging device acquiring the angiographic projection images is taken into account and/or a time-optimized acquisition order of the second angiographic projection images is determined. Mechanical limits of the imaging device can also limit available acquisition geometries, which is advantageously considered when defining additional acquisition geometries. Additionally, the order of the planned scenes/acquisitions can be optimized, for example to minimize the accumulated movement distance of the imaging device and/or the time needed to acquire the second angiographic projection images, such that, in summary, the clinical workflow is improved.

Preferably, when acquiring second angiographic projection images, the image device is automatically or assistedly positioned to realize the additional acquisition geometries. Acquisition parameters describing the additional acquisition geometries may automatically be communicated to the imaging device such that these additional acquisition geometries can be automatically or at least semi-automatically set by moving respective components of the imaging device. If multiple second angiographic projection images are to be acquired or a scan, for example using a rotation axis, is planned, which covers an angular range more or less than with images, only one acquisition process is necessary. The movement of the components of the imaging device takes place in the time between measurements, such that arbitrary acquisition parameters can be connected using a single path of the acquisition arrangement of the imaging device.

In a preferred embodiment, the angiographic projection images are acquired using ECG-triggering and/or collimation to a region of interest comprising the vessel segment, derived from the three-dimensional preliminary information. The second angiographical projection images can be acquired in a certain phase of the cardiac cycle, in particular corresponding to the phase of first angiographic projection images to be used in the reconstruction. In this manner, the radiation dose for the patient can be further reduced since only one or a few frames have to be acquired for each acquisition geometry. Regarding collimation, the spatial extent of the vessel segment may be known from the three-dimensional preliminary information, such that, for each additional acquisition geometry, acquisition parameters defining the collimation such that the vessel segment is shown in the second angiographic projection images can be defined, further advantageously using the preliminary information and reducing the radiation exposure for the patient.

The tree-dimensional reconstruction, i.e. the determination of the reconstruction data set, can also be assisted using the three-dimensional preliminary information, further or even completely automatizing the process. Preferably, at least one segmentation step during reconstruction of the reconstruction data set is performed using the three-dimensional preliminary information. The vessel segment can be localized more simply in the newly acquired second, but also the existing first two-dimensional angiographic projection images, for example by forward projecting the three-dimensional preliminary information. In this manner, an automatic or semi-automatic two-dimensional segmentation of the vessel segment can be facilitated.

It is also preferred if at least a part of the angiographic projection images are geometrically calibrated to each other by 2D-3D-registering the images to the three-dimensional preliminary information. Using such registration, an automated geometric calibration between the different views can be realized, like for example described in US 2017/0018116 A1.

In the preferred embodiment, the method is performed iteratively, using the reconstruction data set as three-dimensional preliminary information. The already improved reconstruction of a first iteration of the inventive method can be used as preliminary information for a further iteration, allowing a further improvement of reconstruction, including the evaluation of the quality of the previous model.

The method according to the invention can be applied to many examination procedures, for example to determine a model for fluid dynamics simulations. In this manner, for example the calculation of decision values, like FFR, can be improved.

The invention further concerns an angiographic imaging device, comprising a control device configured to perform a method according to the invention. All remarks and features pertaining to the method of the invention can also be applied respectively to the imaging device, such that the same advantageous are accomplished.

A computer program according to the invention can be loaded into a storage of a computing device and is configured to perform the steps of a method according to the invention when the computer program is executed on the computing device. The computing device may be, for example, a control device of an angiographic imaging device. The computer program may be stored on an electronically readable storage medium according to the invention, which does include control information comprising a computer program according to the invention, such that when the storage medium is used in a computing device, the steps of a method according to the invention can be performed by executing the computer program. The electronically readable storage medium according to the invention may be a non-transient medium, for example a CD-ROM.

Further details and advantages of the invention can be derived from the following description of preferred embodiments, taken in conjunction with the drawings, in which
- Fig. 1: shows a flowchart of an embodiment of a method according to the invention,
- Fig. 2: is an illustration of possible three-dimensional preliminary information,
- Fig. 3: shows a flowchart of the determination of an overlap measure, and
- Fig. 4: shows a principle drawing of an imaging device according to the invention.

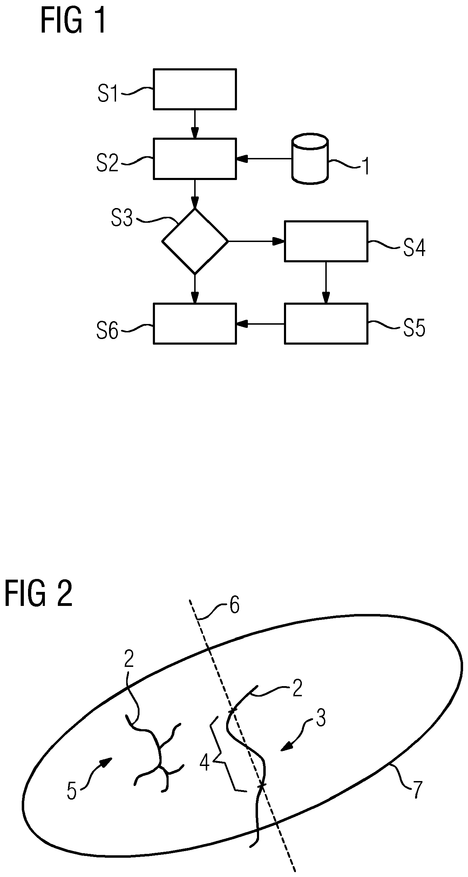

Fig. 1 shows a flowchart of an embodiment of a method according to the invention. In a step S1, at least one, preferably more, first two-dimensional angiographic projection images are acquired using an imaging device, in this case an x-ray device having a c-arm and being adapted for angiographic use. The acquisition geometry of the at least one first angiographic projection image showing a vessel segment of interest is determined by a user, in particular medical staff. Usually, the at least one first angiographic projection image will have a projection direction essentially perpendicular to the course of the vessel segment of interest, which is essentially located isocentric.

The method explained in the following aims at providing a three-dimensional reconstruction data set of the vessel segment, which can, for example, be used as a three-dimensional model for computational fluid dynamics to calculate a FFR value.

In a step S2, the first angiographic projection images, in particular their acquisition geometries, are evaluated regarding their suitability for the reconstruction process. In general, at least one evaluation measure for each first angiographic projection image and/or pairs or multiple first angiographic projection images is calculated using three-dimensional preliminary information 1 at least on the vessel segment, in this case providing information on the position and orientation of the vessel segment in three dimensions as well as information on other vessels in the vessel tree of the vessel segment.

According to embodiments, the three-dimensional preliminary information 1 can be obtained from different sources. Generally, this initial preliminary information on the vessel segment needs only be approximate, meaning a detailed information on the shape of the vessel segment is not necessary, but only the position and orientation of the vessel segment in space.

While the three-dimensional preliminary information 1 can be derived from a proceeding acquisition of the vessel tree of the patient, for example a CT data set or other image data set, which is registered to the imaging device using 2D-3D-registration, the three-dimensional preliminary information can also be based on an initial 3D reconstruction. This initial 3D reconstruction is based on at least two angiographic projection images which are either acquired specifically for this purpose, for example using so-called standard projections or heart sweeps, but preferably at least comprise first angiographic projection images to minimize the radiation exposure of the patient. The used angiographic projection images need not fulfil the requirements for a sufficiently accurate three-dimensional reconstruction, since it is, as discussed above, sufficient to get approximate information on the position and orientation of the vessel segment (and preferably for the vessels of the vessel tree) in 3D space.

It is noted that three-dimensional preliminary information 1 can also be derived if only one first two-dimensional angiographic projection image or scene is available, by using assumptions as for example those laid out above, that the projection direction is perpendicular to the course of the vessel segment and that the vessel segment is placed close or in the isocenter of the angiographic imaging device.

Fig. 2 shows an example of such three-dimensional preliminary information 1, comprising approximate centerlines 2 of the vessel 3 in which the vessel segment 4 is defined and of other vessels 5 in the vessel tree, in this case only roughly indicated. The course of the vessel segment 4 can be approximately described by a straight line 6. Optimal projection directions are perpendicular to the straight line 6, for example along a circular trajectory 7 using the straight line 6 as rotation axis.

For the evaluation in step S2, multiple factors are taken into account. In general, the three-dimensional preliminary information 1 as preliminary knowledge is used to assess the quality and suitability of the first angiographic projection images regarding the three-dimensional reconstruction of the reconstruction data set. The factors taken into account by one or more evaluation measures are the amount of foreshortening, i.e. the amount of perpendicularity of the projection direction to the straight line 6, the difference in projection angles in a pair or group of first angiographic projection images, a potential eccentricity of the vessel segment and the presence and depiction of bifurcations in the vessel segment. Additionally, of course, the curvature of the vessel segment 4 can be taken into account further than just defining a straight line 6, for example by minimizing the amount of foreshortening occurring along the length of the vessel segment 4.

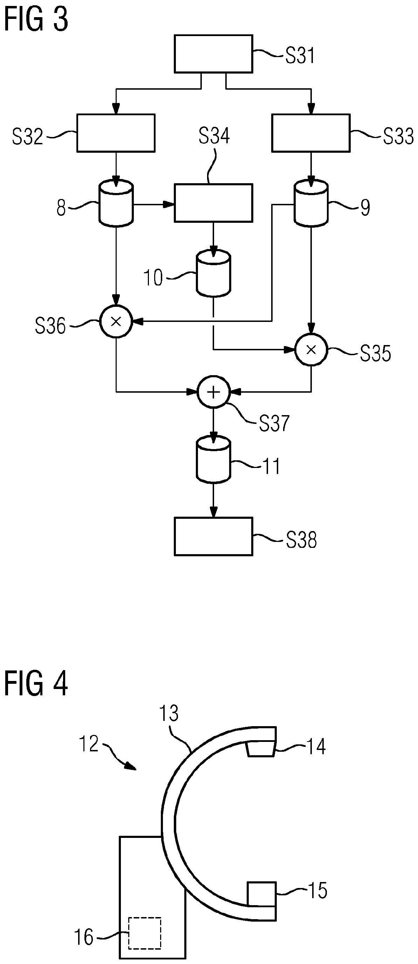

Another important factor in evaluating the first angiographic projection images is the overlap of other vessels 5 of the vessel tree onto the depiction of the vessel segment 4. To take this factor into account, an overlap measure is determined for the acquisition geometry of the respective first angiographic projection image. A general way of determining such an overlap measure (overlap metric) for an acquisition geometry defined relative to the three-dimensional preliminary information 1 is shown in the flowchart of Fig. 3.

In the step S31, if not yet done before, the vessel segment 4 is segmented in the three-dimensional preliminary information 1, preferably in a binarized form. This step can be assisted by a user, for example by marking the vessel segment 4 by two clicks along the centerline 2. In the case of the method according to Fig. 1, however, this correspondence will likely be already known, so that the step S31 has already been performed earlier. The segmentation of the vessel segment 4 allows distinguishing between it and other vessels 5 of the vessel tree, which can be termed as "background information" opposed to the "foreground information" of the vessel segment 4. In steps S32 and S33, first and second virtual projection images 8, 9 are determined, by forward projecting the foreground information/background information in the acquisition geometry, respectively.

It is noted that the virtual projection images 8, 9 are preferably binary images and can be confined to subimages showing the vessel segment 4 and its vicinity.

In a step S34, a distance weighing image 10 is derived from the first virtual projection image 8 by detecting the boundary of the vessel segment 4 in the first virtual projection image 8 and assigning weights decreasing with distance from the boundary to pixels outside the boundary.

Thus, in a step S35, by multiplying the distance weighing image 10 and the second virtual projection image 9 pixel by pixel, a first multiplication result image can be obtained describing prominently other vessels 5 depicted in close vicinity to the vessel segment 4.

In a step S36, the first virtual projection image 8 and the second virtual projection image 9 are also multiplied pixel by pixel to result in a second multiplication result image depicting actually overlapping vessels 5.

Both multiplication result images can be added in a step S37, wherein the addition may be performed weighted. The image values of the summary result image 11 may then be added up in a step S38 to determine the overlap measure. Preferably, the overlap measure is normalized by dividing the sum of the pixel values of summary result image 11 by a sum of the pixel values of first virtual projection image 8.

Such an overlap measure is also calculated for each first angiographic projection image and contributes to the evaluation measure.

The evaluation measure is evaluated in a suitability criterion, which, when fulfilled, selects respective first angiographic projection images for reconstruction of the reconstruction data set; however, the evaluation measures are also evaluated by a quality criterion deciding whether enough sufficiently suitable first angiographic projection images exist to achieve a certain reconstruction quality. The quality criterion is checked in step S3 (Fig. 1).

If the quality criterion is fulfilled, reconstruction of the reconstruction data set can be started in step S6 using the first angiographic projection images fulfilling the suitability criterion. If, however, the quality criterion is not fulfilled in step S3, additional second angiographic projection images are to be acquired.

In a step S4, the three-dimensional preliminary information 1 is used again to determine suitable additional acquisition geometries for the second angiographic projection images. For example, the straight line 6 can be used as axis of rotation or to define an axis of rotation, which in turn defines a circular geometry. Acquisition geometries along this trajectory, for example the trajectory 7 of Fig. 2, can be evaluated using the evaluation measures described above, in particular taking into account the overlap measure. Additionally, limitations in the movement of components of the angiographic imaging device are taken into account when selecting the additional acquisition geometries. The order of using the additional acquisition geometries is optimized to result in minimal acquisition times and/or movement distances.

In a step S5, the planned additional acquisition geometries are transferred to the angiographic imaging device, if not already determined there, such that they can be realized semi-automatically or automatically. The acquisition of the second angiographic projection images may be performed using ECG-triggering. The acquisition parameters defining the additional acquisition geometry also comprise parameters describing the collimation onto the vessel segment 4, again derived from the three-dimensional preliminary information 1.

In step S6, reconstruction of the reconstruction data set is performed using suitable first angiographic projection images and/or second angiographic projection images. The reconstruction is also assisted by using the three-dimensional preliminary information 1. Firstly, the preliminary information 1 is used to localize the vessel segment 4 during segmentation of the vessel segment 4, secondly, an automatic geometric calibration is performed by 2D-3D-registering the three-dimensional preliminary information 1 to the angiographic projection images used for reconstruction.

It is noted that the method described here can also be performed iteratively by using the resulting reconstruction data set of a first pass as three-dimensional preliminary information 1 for a second pass of the method.

Fig. 4 shows a principle drawing of an angiographic imaging device 12 according to the invention. The angiographic imaging device 12 comprises a c-arm 13 having an x-ray detector 14 and an x-ray source 15 mounted to opposing ends. The c-arm 13 and/or the components 14, 15 may be moveable to realize certain acquisition geometries.

The x-ray imaging device 12 further comprises a control device 16 configured to perform a method according to the invention. The control device 16 can thus comprise an acquisition unit for performing acquisition in determined or chosen acquisition geometries, an evaluation unit for determining evaluation measures, a quality criterion evaluation unit for evaluating the quality criterion, a determination unit for determining additional acquisition geometries and a reconstruction unit for reconstructing the reconstruction data set.

Although the present invention has been described in detail with reference to the preferred embodiment, the present invention is not limited by the disclosed examples from which the skilled person is able to derive other variations without departing from the scope of the invention, which is defined by the appended claims.

## Claims

1. Method for at least symbolically reconstructing a reconstruction data set of at least one vessel segment (4) in a vessel tree of a patient, wherein input data for the reconstruction comprises at least two two-dimensional angiographic projection images taken in different acquisition geometries, the method comprising:
- acquiring at least one first angiographic projection image showing the vessel segment (4),
- automatically determining an evaluation measure for each first angiographic projection image using a three-dimensional preliminary information (1) on the vessel segment (4), the evaluation measure describing the suitability of the at least one angiographic projection image for reconstructing the reconstruction data set,
wherein the evaluation measure for one first angiographic projection image is determined depending on foreshortening of the vessel segment (4) and/or overlap with other vessels (5) occurring in the acquisition geometry of the corresponding first angiographic projection image,
- if a quality criterion evaluating the evaluation measure is not fulfilled, determining at least one additional acquisition geometry using the three-dimensional preliminary information (1) and/or the evaluation measure and acquiring, in each additional acquisition geometry, at least one second angiographic projection image,
- reconstructing the reconstruction data set from the at least one second angiographic projection image and/or at least one of the at least one first angiographic projection image fulfilling a suitability criterion evaluating the evaluation measure.

2. Method according to claim 1, **characterized in that** the three-dimensional preliminary information (1) comprises or is derived from a previously acquired three-dimensional image data set, in particular a CT data set, which is registered to the imaging device (12) acquiring the angiographic projection images, and/or the three-dimensional preliminary information (1) is determined by preliminary reconstruction from, in particular the at least two first, angiographic projection images, and/or the three-dimensional preliminary information (1) is derived one two-dimensional first angiographic projection image and at least one assumption on how the acquisition geometry of the first angiographic projection image was chosen.

3. Method according to one of the preceding claims, **characterized in that** a foreshortening value is determined by comparing a direction of the vessel segment (4) according to the three-dimensional preliminary information (1) and an image plane according to the acquisition geometry of the first angiographic projection image.

4. Method according to one of the preceding claims, **characterized in that** the evaluation measure for a pair of first angiographic projection images is determined depending on the relative projection angle of the acquisition geometries.

5. Method according to one of the preceding claims, **characterized in that** to determine the evaluation measure and/or the additional acquisition geometry an overlap measure for an acquisition geometry is calculated by
- forward projecting the three-dimensional preliminary information (1) to determine a first virtual projection image (8) relating to the vessel segment (4) and a second virtual projection image (9) relating to other vessels (5) in the vessel tree,
- deriving a distance weighing image (10) from the first virtual projection image (8), comprising a weight decreasing outward from a boundary of the vessel segment (4) in the first virtual projection image (8),
- measuring a proximity of other vessels (5) by multiplying the distance weighing image (10) and the second virtual projection image (9) and measuring an overlap by multiplying the first virtual projection image (8) and the second virtual projection image (9),
- deriving the overlap measure by combining the multiplication results.

6. Method according to one of the preceding claims, **characterized in that** to determine the evaluation measure and/or the additional acquisition geometry at least one eccentricity of the vessel segment (4) and/or the visibility of at least one bifurcation in the vessel segment (4) are taken into account.

7. Method according to one of the preceding claims, **characterized in that**, when determining additional acquisition geometries, also the movement range of the imaging device (12) acquiring the angiographic projection images is taken into account and/or a time-optimized acquisition order of the second angiographic projection images is determined and/or, when acquiring second angiographic projection images, the imaging device (12) is automatically or assistedly positioned to realize the additional acquisition geometries.

8. Method according to one of the preceding claims, **characterized in that** the angiographic projection images are acquired using ECG-triggering and/or collimation to a region of interest comprising the vessel segment (4), derived from the three-dimensional preliminary information (1).

9. Method according to one of the preceding claims, **characterized in that** at least one segmentation step during reconstruction of the reconstruction data set is performed using the three-dimensional preliminary information (1).

10. Method according to one of the preceding claims, **characterized in that** at least a part of the angiographic projection images are geometrically calibrated to each other by 2D-3D-registering the images to the three-dimensional preliminary information (1).

11. Method according to one of the preceding claims, **characterized in that** the method is performed iteratively, using the reconstruction data set as three-dimensional preliminary information (1).

12. Angiographic imaging device (12), comprising a control device (16) configured to perform a method according to one of the preceding claims.

13. Computer program, configured to perform the steps of a method according to one of the claims 1 to 11 when the computer program is executed on a computing device.

14. Electronically readable storage medium, on which a computer program according to claim 13 is stored.

## Patentansprüche

1. Verfahren zur mindestens symbolischen Rekonstruktion eines Rekonstruktionsdatensatzes von mindestens einem Gefäßsegment (4) in einem Gefäßbaum eines Patienten, wobei Eingabedaten für die Rekonstruktion mindestens zwei zweidimensionale angiographische Projektionsbilder umfassen, die in unterschiedlichen Erfassungsgeometrien aufgenommen wurden, wobei das Verfahren umfasst:
- Erfassen von mindestens einem ersten angiographischen Projektionsbild, welches das Gefäßsegment (4) zeigt,
- automatisches Ermitteln eines Auswertungsmaßes für jedes erste angiographische Projektionsbild unter Verwendung von dreidimensionalen vorläufigen Informationen (1) zu dem Gefäßsegment (4),
wobei das Auswertungsmaß die Eignung des mindestens einen angiographischen Projektionsbilds zur Rekonstruktion des Rekonstruktionsdatensatzes beschreibt, wobei das Auswertungsmaß für ein erstes angiographisches Projektionsbild in Abhängigkeit von perspektivischem Verkürzen des Gefäßsegments (4) und/oder Überlappen mit anderen Gefäßen (5) ermittelt wird, die in der Erfassungsgeometrie des entsprechenden ersten angiographischen Projektionsbildes stattfinden,
- falls ein Qualitätskriterium, welches das Auswertungsmaß auswertet, nicht erfüllt ist, Ermitteln von mindestens einer zusätzlichen Erfassungsgeometrie unter Verwendung der dreidimensionalen vorläufigen Informationen (1) und/oder des Auswertungsmaßes, und Erfassen von mindestens einem zweiten angiographischen Projektionsbild in jeder zusätzlichen Erfassungsgeometrie,
- Rekonstruieren des Rekonstruktionsdatensatzes aus dem mindestens einen zweiten angiographischen Projektionsbild und/oder mindestens einem von dem mindestens einen ersten angiographischen Projektionsbild, welches ein Eignungskriterium erfüllt, welches das Auswertungsmaß auswertet.

2. Verfahren nach Anspruch 1, **dadurch gekennzeichnet, dass** die dreidimensionalen vorläufigen Informationen (1) einen zuvor erfassten dreidimensionalen Bilddatensatz, insbesondere einen CT-Datensatz, umfassen oder davon abgeleitet sind, welcher auf der Bildgebungsvorrichtung (12) registriert ist, welche die angiographischen Projektionsbilder erfasst, und/oder die dreidimensionalen vorläufigen Informationen (1) durch vorläufige Rekonstruktion aus insbesondere den mindestens zwei ersten angiographischen Projektionsbildern ermittelt werden, und/oder die dreidimensionalen vorläufigen Informationen (1) von einem zweidimensionalen ersten angiographischen Projektionsbild und mindestens einer Annahme abgeleitet werden, wie die Erfassungsgeometrie des ersten angiographischen Projektionsbilds gewählt wurde.

3. Verfahren nach einem der vorhergehenden Ansprüche, **dadurch gekennzeichnet, dass** ein perspektivischer Verkürzungswert ermittelt wird, indem eine Richtung des Gefäßsegments (4) gemäß den dreidimensionalen vorläufigen Informationen (1) und eine Bildebene gemäß der Erfassungsgeometrie des ersten angiographischen Projektionsbilds verglichen werden.

4. Verfahren nach einem der vorhergehenden Ansprüche, **dadurch gekennzeichnet, dass** das Auswertungsmaß für ein Paar von ersten angiographischen Projektionsbildern in Abhängigkeit von dem relativen Projektionswinkel der Erfassungsgeometrien ermittelt wird.

5. Verfahren nach einem der vorhergehenden Ansprüche, **dadurch gekennzeichnet, dass** zum Ermitteln des Auswertungsmaßes und/oder der zusätzlichen Erfassungsgeometrie ein Überlappungsmaß für eine Erfassungsgeometrie berechnet wird durch:
- Vorwärtsprojektion der dreidimensionalen vorläufigen Informationen (1), um ein erstes virtuelles Projektionsbild (8) in Bezug auf das Gefäßsegment (4) und ein zweites virtuelles Projektionsbild (9) in Bezug auf andere Gefäße (5) in dem Gefäßbaum zu ermitteln,
- Ableiten eines Distanzwichtungsbildes (10) aus dem ersten virtuellen Projektionsbild (8), das eine Wichtung umfasst, die in dem ersten virtuellen Projektionsbild (8) von einer Grenze des Gefäßsegments (4) auswärts abnimmt,
- Messen einer Nähe zu anderen Gefäßen (5), indem das Distanzwichtungsbild (10) und das zweite virtuelle Projektionsbild (9) multipliziert werden und eine Überlappung gemessen wird, indem das erste virtuelle Projektionsbild (8) und das zweite virtuelle Projektionsbild (9) multipliziert werden,
- Ableiten des Überlappungsmaßes, indem die Multiplikationsergebnisse kombiniert werden.

6. Verfahren nach einem der vorhergehenden Ansprüche, **dadurch gekennzeichnet, dass** zum Ermitteln des Auswertungsmaßes und/oder der zusätzlichen Erfassungsgeometrie mindestens eine Exzentrizität des Gefäßsegments (4) und/oder die Sichtbarkeit von mindestens einer Bifurkation in dem Gefäßsegment (4) berücksichtigt werden.

7. Verfahren nach einem der vorhergehenden Ansprüche, **dadurch gekennzeichnet, dass**, wenn zusätzliche Erfassungsgeometrien ermittelt werden, auch der Bewegungsbereich der Bildgebungsvorrichtung (12), die die angiographischen Projektionsbilder erfasst, berücksichtigt wird und/oder eine zeitoptimierte Erfassungsreihenfolge der zweiten angiographischen Projektionsbilder ermittelt wird, und/oder, wenn zweite angiographische Projektionsbilder erfasst werden, die Bildgebungsvorrichtung (12) automatisch oder unterstützt positioniert wird, um die zusätzlichen Erfassungsgeometrien zu realisieren.

8. Verfahren nach einem der vorhergehenden Ansprüche, **dadurch gekennzeichnet, dass** die angiographischen Projektionsbilder unter Verwendung von EKG-Auslösung und/oder Kollimation auf eine interessierende Region erfasst werden, welche das Gefäßsegment (4) umfasst, abgeleitet von den dreidimensionalen vorläufigen Informationen (1).

9. Verfahren nach einem der vorhergehenden Ansprüche, **dadurch gekennzeichnet, dass** mindestens ein Segmentierungsschritt während der Rekonstruktion des Rekonstruktionsdatensatzes unter Verwendung der dreidimensionalen vorläufigen Informationen (1) durchgeführt wird.

10. Verfahren nach einem der vorhergehenden Ansprüche, **dadurch gekennzeichnet, dass** mindestens ein Teil der angiographischen Projektionsbilder durch 2D-3D-Registrierung der Bilder mit den dreidimensionalen vorläufigen Informationen (1) geometrisch aufeinander kalibriert werden.

11. Verfahren nach einem der vorhergehenden Ansprüche, **dadurch gekennzeichnet, dass** das Verfahren iterativ unter Verwendung des Rekonstruktionsdatensatzes als dreidimensionale vorläufige Informationen (1) durchgeführt wird.

12. Angiographische Bildgebungsvorrichtung (12), umfassend eine Steuerungsvorrichtung (16), die ausgestaltet ist, um ein Verfahren gemäß einem der vorhergehenden Ansprüche durchzuführen.

13. Computerprogramm, das zur Durchführung der Schritte eines Verfahrens gemäß einem der Ansprüche 1 bis 11 ausgestaltet ist, wenn das Computerprogramm auf einer Rechenvorrichtung ausgeführt wird.

14. Elektronisch lesbares Speichermedium, auf dem ein Computerprogramm gemäß Anspruch 13 gespeichert ist.

## Revendications

1. Procédé pour reconstruire, au moins symboliquement, un jeu de données de reconstruction d'au moins un segment (4) de vaisseau dans un arbre de vaisseau d'un patient, dans lequel une donnée d'entrée pour la reconstruction comprend au moins deux images de projection angiographiques à deux dimensions prises dans des géométries d'acquisition différentes,
le procédé comprenant :
- l'acquisition d'au moins une première image de projection angiographique montrant le segment (4) de vaisseau,
- la détermination automatique d'une mesure d'évaluation pour chaque première image de projection angiographique, en utilisant une information (1) préliminaire à trois dimensions sur le segment (4) de vaisseau, la mesure d'évaluation décrivant la convenance de la au moins une image de projection angiographique pour reconstruire le jeu de données de reconstruction,
dans lequel on détermine la mesure d'évaluation pour une première image de projection angiographique en fonction du raccourci du segment (4) de vaisseau et/ou du chevauchement avec d'autres vaisseaux (5) se produisant dans la géométrie d'acquisition de la première image de projection angiographique correspondante,
- si un critère de qualité évaluant la mesure d'évaluation n'est pas satisfait, on détermine au moins une géométrie d'acquisition supplémentaire en utilisant l'information (1) préliminaire à trois dimensions et/ou la mesure d'évaluation et on acquiert dans chaque géométrie d'acquisition supplémentaire au moins une deuxième image de projection géographique,
- on reconstruit le jeu de données de reconstruction à partir de la au moins une deuxième image de projection géographique et/ou d'au moins l'une de la au moins une première image de projection géographique satisfaisant un critère de convenance évaluant la mesure d'évaluation.

2. Procédé suivant la revendication 1, **caractérisé en ce que** l'information (1) préliminaire à trois dimensions comprend un jeu de données d'image à trois dimensions acquis précédemment ou s'en déduit, en particulier d'un jeu de données CT, qui est enregistré dans le dispositif (12) d'imagerie acquérant les images de projection angiographiques et/ou on détermine l'information (1) préliminaire à trois dimensions par reconstruction préliminaire à partir, en particulier, des au moins deux premières images de projection angiographiques et/ou on déduit l'information (1) préliminaire à trois dimensions d'une première image de projection angiographique à deux dimensions et d'au moins une hypothèse sur la manière dont la géométrie d'acquisition de la première image de projection angiographique a été choisie.

3. Procédé suivant l'une des revendications précédentes, **caractérisé en ce que** l'on détermine une valeur de raccourci en comparant une direction du segment (4) de vaisseau, suivant l'information (1) préliminaire à trois dimensions, à un plan image suivant la géométrie d'acquisition de la première image de projection angiographique.

4. Procédé suivant l'une des revendications précédentes, **caractérisé en ce que** l'on détermine la mesure d'évaluation pour une paire de premières images de projection angiographiques en fonction de l'angle de projection relatif des géométries d'acquisition.

5. Procédé suivant l'une des revendications précédentes, **caractérisé en ce que** pour déterminer la mesure d'évaluation et/ou la géométrie d'acquisition supplémentaire, on calcule une mesure de chevauchement pour une géométrie d'acquisition en
- projetant vers l'avant l'information (1) préliminaire à trois dimensions pour déterminer une première image (8) de projection virtuelle se rapportant au segment (4) de vaisseau et une deuxième image (9) de projection virtuelle se rapportant à d'autres vaisseaux (5) de l'arbre de vaisseau,
- en déduisant une image (10) de pondération de distance à partir de la première image (8) de projection virtuelle, comprenant une diminution de pondération vers l'extérieur à partir d'une limite du segment (4) de vaisseau dans la première image (8) de projection virtuelle,
- en mesurant une proximité d'autres vaisseaux (5) en multipliant l'image (10) de pondération de distance par la deuxième image (9) de projection virtuelle et en mesurant un chevauchement en multipliant la première image (8) de projection virtuelle par la deuxième image (9) de projection virtuelle,
- en déduisant la mesure de chevauchement en combinant les résultats de multiplication.

6. Procédé suivant l'une des revendications précédentes, **caractérisé en ce que** pour déterminer la mesure d'évaluation et/ou la géométrie d'acquisition supplémentaire, on prend en considération au moins une excentricité du segment (4) de vaisseau et/ou la visibilité d'au moins une bifurcation du segment (4) de vaisseau.

7. Procédé suivant l'une des revendications précédentes, **caractérisé en ce que**, lorsque l'on détermine des géométries d'acquisition supplémentaires, on prend en considération aussi l'intervalle de déplacement du dispositif (12) d'imagerie acquérant les images de projection angiographiques et/ou on détermine un ordre d'acquisition optimisé en fonction du temps des deuxièmes images de projection angiographiques et/ou lorsque l'on acquiert des deuxièmes images de projection angiographiques, on met, de manière automatique ou avec assistance, en position le dispositif (12) d'imagerie pour réaliser les géométries d'acquisition supplémentaires.

8. Procédé suivant l'une des revendications précédentes, **caractérisé en ce que** l'on acquiert les images de projection angiographiques en utilisant un déclenchement ECG et/ou une collimation sur une région à laquelle on s'intéresse comprenant le segment (4) de vaisseau déduite de l'information (1) préliminaire à trois dimensions.

9. Procédé suivant l'une des revendications précédentes, **caractérisé en ce que** l'on effectue au moins un stade de segmentation pendant la reconstruction du jeu de données de reconstruction, en utilisant l'information (1) préliminaire à trois dimensions.

10. Procédé suivant l'une des revendications précédentes, **caractérisé en ce qu'**au moins une partie des images de projection angiographiques sont étalonnées géométriquement les unes aux autres par un enregistrement en 2D-3D des images pour l'information (1) préliminaire à trois dimensions.

11. Procédé suivant l'une des revendications précédentes, **caractérisé en ce que** l'on effectue le procédé par itération, en utilisant le jeu de données de reconstruction comme information (1) préliminaire à trois dimensions.

12. Dispositif (12) d'imagerie angiographique, comprenant un dispositif (16) de commande, configuré pour effectuer un procédé suivant l'une des revendications précédentes.

13. Programme d'ordinateur configuré pour effectuer les stades d'un procédé suivant l'une des revendications 1 à 11 lorsque le programme est exécuté sur un dispositif informatique.

14. Support de mémoire déchiffrable électroniquement, sur lequel un programme d'ordinateur suivant la revendication 13 est mis en mémoire.
